# EUROPEAN PATENT SPECIFICATION

(11) **EP 0 891 224 B1**
(45) Date of publication and mention of the grant of the patent: **24.11.1999**
(21) Application number: 97909050.3
(22) Date of filing: 18.03.1997
(51) Int. Cl.: B01J 23/52, B01J 37/02, C07C 67/055

(54) **A METHOD OF PREPARING A VINYL ACETATE CATALYST EMPLOYING AN ALKALI METAL BORATE**
VERFAHREN ZUR HERSTELLUNG EINES VINYLACETATKATALYSATORS UNTER VERWENDUNG EINES ALKALIMETALLBORATS
PROCEDE DE PREPARATION DE CATALYSEUR POUR L'ACETATE DE VINYLE AU MOYEN DE BORATE DE METAL ALCALIN

(30) Priority: 01.04.1996 US 627791
(43) Date of publication of application: 20.01.1999
(73) Proprietor: Hoechst Celanese Corporation, Warren, NJ 07059 (US)
(72) Inventor: NICOLAU, Ioan, Corpus Christi, TX 78413 (US); COLLING, Philip, M., Corpus Christi, TX 78411 (US)
(74) Representative: De Minvielle-Devaux, Ian Benedict Peter
(86) International application number: US9704324
(87) International publication number: WO9736678

(56) References cited:
- EP-A- 0 563 414
- EP-A- 0 634 209
- WO-A-94/08714

## Description

### BACKGROUND OF THE INVENTION

The present invention relates to a novel method of producing a catalyst useful for producing unsaturated esters by gas phase reaction. In particular, the present invention is directed to a novel method of producing a catalyst useful in the gas phase formation of vinyl acetate from the reaction of ethylene, oxygen and acetic acid.

It is known in the art to produce vinyl acetate by reacting ethylene, oxygen and acetic acid in a gaseous phase and in the presence of a catalyst comprising palladium, gold and an alkali metal acetate supported on certain carrier materials such as silica. Such catalyst systems can exhibit a high activity. Unfortunately, results utilizing such palladium and gold catalysts have been inconsistent. This inconsistency appears to be based somewhat on the distribution pattern or profile of the catalyst components which are deposited on and in relation to the carrier. For example, when use is made of known vinyl acetate catalyst systems comprising a porous support with palladium and gold, the metal components deposited at or about the carrier interiors or central regions do not always contribute significantly to reaction mechanisms, since reactants are not readily able to diffuse into the central or inner regions of the porous network of the catalyst. More importantly, products of catalyst synthesis formed in the catalyst interior must diffuse from the interior outward, again coming into contact with the active phase in the outer region of the catalyst. Consequently, these interior-formed products undergo further reaction and are often converted to unuseful by-products. The most effective reactions occur when the catalytic metal is formed as a thin shell on the surface regions of the catalyst as diffusion of reactants and products can be readily achieved to provide good product yields and reduced by-product formulation.

Various patents have been granted based on the desire to more evenly distribute and anchor the gold and palladium catalytic components within a narrow band on the carrier surface to provide a vinyl acetate catalyst having high yield, good selectivity and long life. Examples of such patents include U.S. Patent Nos. 4,087,622; 4,048,096; 3,822,308; 3,775,342 and British Patent 1,521,652.

The basic method of forming the vinyl acetate catalyst containing palladium and gold deposited on a catalyst carrier comprises (1) impregnating the carrier with aqueous solutions of water-soluble palladium and gold compounds, (2) precipitating water-insoluble palladium and gold compounds on the catalyst carrier by contacting the impregnated catalyst carrier with a solution of compounds capable of reacting with the water-soluble palladium and gold compounds to form the water-insoluble precious metal compounds (3) washing the treated carrier with water to remove anions which are freed from the initially impregnated palladium and gold compounds during precipitation and (4) converting the water-insoluble palladium and gold compounds to the free metal by treatment with a reducing agent. A final treatment usually involves (5) impregnating the reduced catalyst with an aqueous alkali metal acetate solution and (6) drying the final catalyst product.

Attempts to provide a uniform distribution of the palladium and gold metals on the carrier has involved manipulation of the above mentioned steps and/or by using carrier materials having various specified pore dimensions. Particularly useful improvements in preparing highly active catalysts for preparing vinyl acetate are described in commonly assigned U.S. Patent Nos. 5,314,858 and 5,332,710. These two patents describe processes for improving palladium and gold distribution on a carrier by manipulating precipitation step (2), the "fixing" of the water soluble precious metal compounds to the carrier as water-insoluble compounds. In U.S. 5,314,858, fixing precious metals on the carrier is achieved utilizing two separate precipitation stages to avoid using large excesses of a "fixing" compound. U.S. 5,332,710 describes fixing the precious metals by rotating impregnated catalyst carriers while the impregnated carriers are immersed in a reaction solution at least during the initial precipitation period. Such roto-immersion procedure has been found to yield catalysts in which the precipitated carrier metals are more evenly distributed in a narrow band on the carrier surface.

Other attempts to improve catalytic activity have involved using catalyst carriers of particular pore size or particular shapes. Catalyst carriers useful for producing vinyl esters are typically composed of silica, alumina, aluminum silicates or spinels. Silica is the preferred carrier material because silica is porous and is a neutral carrier for precious metal deposition. The carriers are usually shaped as spheres, tablets or cylinders. Spherical shaped carriers having diameters in the range of 4-8 mm often are employed.

As the catalytic activity increases it is preferable, for the purpose of producing an unsaturated ester on an industrial scale, to increase the raw material gas volume comprised of olefin, organic carboxylic acid and oxygen passing across the catalyst. Catalytic activity is usually evaluated by space time yield (STY). One reason to increase raw material gas volume passing across a catalyst is to prevent formation of hot spots on the active catalyst. Since formation reactions of unsaturated esters are exothermic, an increase in catalytic activity can excessively heat portions of the catalyst. Inefficient heat distribution on a catalyst undesirably leads to side reactions such as formation of carbon dioxide which results in less selectivity for formation of the unsaturated ester such as vinyl acetate.

Another problem associated with increasing activity of the vinyl ester catalysts is the production of heavy ends during vinyl ester synthesis. Heavy ends are by-product residues which comprise high molecular weight organic compounds formed during unsaturated ester synthesis. Such heavy ends include, but are not limited to, ethylidene diacetate; 1,1-diacetoxy ethylene; cis and trans-diacetoxy ethylene; ethylene glycol diacetate; vinyl acetoxyacetate; vinyl acetoxyacetic acid; ethylene glycol monoacetate; and cyclopropane carboxylic acid. Unsaturated ester synthesis, such as vinyl acetate synthesis, can often produce a heavy ends selectivity of up to about 2% based on the reactant ethylene. The heavy ends can be readily removed and separated from the desired product by distillation, and the bottoms containing the heavy ends collected and disposed of by waste site dumping or burning. However, disposal of the heavy ends by such methods is not practical. The heavy ends are considered toxic or the burning thereof can cause formation and release of toxic products into the environment. Pollution laws and guidelines in many areas of the world strictly limit the ability to dump toxic solid waste or to burn same. Thus, any reduction in heavy ends formation during unsaturated ester synthesis is very desirable in order to reduce waste disposal costs. There is a need, therefore, for a method of preparing an active catalyst that can be employed in the synthesis of unsaturated esters and that results in reduced heavy ends formation.

Accordingly, it is an object of the present invention to provide a method of preparing a catalyst which has reduced heavy ends during vinyl ester synthesis.

A further object of the present invention is to provide a method of preparing a catalyst for the selective synthesis of vinyl acetate.

Another object of the present invention is to provide a method of preparing a catalyst which has high activity for vinyl acetate synthesis.

Still yet a further object of the present invention is to provide a method of making a vinyl ester catalyst which has a long life.

Other objects and advantages of the present invention are set forth in the description which follows and will become apparent to a person of skill in the art upon practicing the present invention.

### SUMMARY OF THE INVENTION

A method has now been found that produces a catalyst containing palladium and gold useful for the production of vinyl esters from ethylene, lower carboxylic acids with 2-4 carbon atoms and oxygen in the gas phase at elevated temperature and at normal or elevated pressure that provides for reduced heavy ends formation during vinyl ester synthesis and accordingly alleviates the need for heavy ends disposal. Disposal of such heavy ends presents environmental pollution problems.

Typically in the preparation of vinyl ester catalysts, water-soluble palladium and gold compounds are fixed to a carrier with an excess of an alkaline compound such as sodium hydroxide or potassium hydroxide to insure that all of the water-soluble precious metal compounds are precipitated as water-insoluble compounds on the catalyst carrier. The fixed precious metal compounds are reduced with a reducing agent such as ethylene or hydrazine to form the precious metals. The resulting catalyst can be employed to synthesize vinyl esters. Such prepared catalysts, although highly active, often yield undesirable levels of heavy ends.

To reduce the production of heavy ends during vinyl ester synthesis and in accordance with the present invention, a useful catalyst is formed by (1) simultaneously or successively impregnating a catalyst carrier with aqueous solutions of water-soluble palladium and gold compounds such as sodium-palladium chloride and auric chloride, (2) fixing the precious metals on the carrier by treatment of the impregnated carrier with an alkali metal borate to precipitate palladium and gold compounds, i.e., hydroxides, on the carrier surface, (3) reducing the precious metal compounds to free palladium and gold, and (4) washing with water to remove the chloride ion (or other anion). By replacing the typical alkali metal hydroxide fix with an alkali metal borate fix, it has been found that the amount of heavy ends produced during the formation of vinyl esters, such as vinyl acetate, by the process of reacting ethylene, lower carboxylic acid and oxygen in the gas phase is reduced, and the catalyst activity is improved. Further, the catalyst can be readily reused without loss of catalytic activity.

### DETAILED DESCRIPTION OF THE INVENTION

In an improved method of preparing a catalyst employed in the synthesis of unsaturated esters, palladium and gold compounds are fixed to the catalyst carrier with an alkali metal borate.

The support material for the catalyst according to the present invention can be of any diverse geometrical shape. For example, the support can be shaped as spheres, tablets or cylinders. The geometrical dimensions of the support material can, in general, be in the range of 1-8 mm. A most suitable geometrical shape is, in particular, the spherical shape, for example, spheres with diameters in the range of 4-8 mm.

The specific surface area of the support material can vary within wide limits. For example, support materials which have an inner surface area of 50-300 m²/g and especially 100-200 m²/g (measured according to BET) are suitable.

Examples of support materials which can be used include silica, aluminum oxide, aluminum silicates or spinels. Silica is the preferred support material.

The catalyst carrier is first impregnated with an aqueous solution containing water-soluble palladium compounds and water-soluble gold compounds. Separate solutions of palladium and gold compounds also can be used successively, but it is less convenient to proceed in such a fashion. Palladium (II) chloride, sodium palladium (II) chloride, potassium palladium (II) chloride, palladium (II) nitrate or palladium (II) sulfate are examples of suitable water-soluble palladium compounds. The sodium or potassium salts of auric (III) chloride or tetrachloroauric (III) acid can be used as the water-soluble gold compounds. Tetrachloroauric (III) acid and sodium palladium (II) chloride are preferred because of their good water solubility. The quantity of these compounds employed is such as to provide about 1 to about 10 grams of palladium and about 0.5 to about 10 grams of gold per liter of finished catalyst. Catalysts containing even higher or lower amounts of the precious metals relative to that recited above also can be useful in the formation of vinyl acetate by reaction of ethylene, oxygen and acetic acid in the vapor phase as long as the catalyst is formed by the novel method set forth herein. The volume of solution used for impregnating the support with the precious metals is important. For effective deposition, the volume of the impregnating solution should be from about 95 to about 100% of the dry absorptive capacity of the catalyst support and preferably it is about 98-99%. Such impregnation technique is characterized as the "incipient wetness" method. Impregnation of the carrier with the water-soluble palladium and gold compounds occurs over a period of about 1 to about 2 hours.

After impregnating the carrier with the water-soluble palladium and the water-soluble gold compounds, the water-soluble precious metal compounds then are fixed to the carrier as water-insoluble precious metal compounds. The fixing solution comprises an aqueous solution of an alkali metal borate. Any suitable alkali metal borate can be employed to practice the present invention. Such alkali metal borates include, but are not limited to, sodium borates such as sodium tetraborate, sodium tetraborate pentahydrate, and sodium tetraborate decahydrate, or potassium borates such as potassium tetraborate, potassium tetraborate decahydrate and potassium tetraborate pentahydrate. It is preferred to use aqueous solutions of sodium tetraborate pentahydrate or sodium tetraborate decahydrate. Treatment with the aqueous borate solution converts the water-soluble precious metal salts to water-insoluble metal hydroxides.

The water-soluble precious metal compounds can be fixed to the carrier with an alkali metal borate as water-insoluble precious metal compounds by any suitable fixing process employed in the art. During the fixing period the pH of the fixing solution can drop from about 12.0 to as low as about 6.0 depending on the duration of the fixing period. Preferably, the pH of the fixing solution ranges from about 6.5 to about 8.0 by completion of fixing to assure optimum fixing conditions. The amount of alkali metal borate used is in excess on a molar basis of that required to react with all of the impregnated water-soluble precious metal compounds. Thus, the molar ratio of alkali metal borate to palladium and gold can range from about 1.5:1 to about 2.5:1, preferably, about 2:1.

One method of fixing the precious metals onto the carrier is the "incipient wetness" method whereby as above described, a specified volume of the fixing solution, e.g., aqueous alkali metal borate, equal to the dry absorbtivity of the carrier is poured onto the porous supports which have been impregnated with the precious metal salts. The treated carriers are allowed to stand until precipitation is complete. Molar ratios of borate salt to the palladium and gold metal are those as expressed above. When fixing is performed by the incipient wetness method, the impregnated carriers are air dried prior to fixing with the aqueous alkali metal borate.

Preferably, fixing is done by a process designated "rotation immersion" which is set forth in U.S. Patent No. 5,332,710 issued July 26, 1994 to Nicolau et al. In this process, the impregnated carrier is immersed in an alkali metal borate fixing solution and tumbled or rotated therein at least during the initial stages of the precipitation of the water-insoluble precious metal compounds. The rotation or tumbling of the carriers in the alkali metal borate fixing solution preferably proceeds for at least about 0.5 hour upon the initial treatment and, most preferably, for at least about 2.5 hours. The rotation immersion treatment can last as long as up to about 4 hours. The treated carrier can be allowed to stand in the fixing solution to insure that complete precipitation of the water-soluble precious metal compounds to water-insoluble compounds takes place.

Any type of rotation or tumbling equipment can be used as the exact apparatus utilized is not critical. What is critical, however, is the extent of the rotating motion. Thus, the rotation preferably is sufficient such that all surfaces of the impregnated carriers are evenly contacted with the alkali metal borate fixing solution. The rotation preferably is not harsh enough such that actual abrasion of the water-insoluble precious metal compounds takes place such that the water-insoluble compounds are abraded off the carrier surface. On the other hand, it has been found or believed that some small extent of abrasion of the water-insoluble precious metal compounds actually works to more evenly distribute the water-insoluble precious metal compounds on the carrier surface. The extent of rotation preferably is about 1 to about 10 rpm and possibly even higher depending upon the exact carrier utilized and the amount of precious metal to be deposited on the carrier. The rpm to be used is variable and also can depend upon the apparatus utilized, the size and shape of the carrier, the type of carrier, metal loadings, etc., but preferably falls within the guidelines expressed above that while a small amount of abrasion can be beneficial, it is not to be such that the water-insoluble compounds are actually abraded off the carrier surface.

The fixing step also can be divided into at least two separate stages of treatment with the alkali metal borate fixing solution. Such a fixing step is disclosed in U.S. Patent No. 5,314,858, issued May 24, 1994 to Colling. In each separate fixing treatment, the amount of the alkali metal borate compound is no more than that equal to the molar amount required to react with all of the precious metal compound which is present on the carrier as a water-soluble compound. No excess of fixing compound is used. The treatment in the second fixing stage can be equivalent to that of the first stage wherein the treated and partially fixed carrier is impregnated with the fixing solution at the desired borate concentration and in a total volume solution again equivalent to the dry absorbtivity of the carrier. The carrier also can be impregnated and fixed in the second fixing stage by the rotation immersion process discussed above.

The fixed precious metal compounds are then treated with a reducing agent in order to convert the precipitated precious metal compounds, e.g., hydroxides, on the carrier into metallic form. The reduction can be carried out in the liquid Phase, for example, with aqueous hydrazine and the hydrates thereof, or in the gas phase, for example, with hydrogen or hydrocarbons, for example, ethylene. Preferably, the reduction step is carried out in the liquid phase with aqueous hydrazine. When the reduction is carried out in the preferred liquid phase with a solution of hydrazine, the reaction preferably is carried out at normal temperature, i.e., about 20-25°C. The molar ratio of hydrazine to palladium and gold metal ranges from about 8:1 to about 15:1, preferably, about 12:1.

The aqueous hydrazine solution can be applied to the carriers by any known method including the incipient wetness and rotation immersion methods described above for the fixing solution. A particularly useful method is to add the aqueous hydrazine solution to the fixed carriers during the later stages of the rotation immersion fixing process. Thus, after rotating the impregnated carriers for about 0.5 to 4 hours in the alkali metal borate solution, the aqueous hydrazine solution is added and the fixed carriers again rotated for about 0.5 to 3 hours at a rate of from about 1 to about 10 rpm. The rotation is stopped and the carriers are allowed to sit for about 2 to about 5 hours to allow completion of the reduction process.

When the reduction step is carried out in the gas phase with ethylene, it is advantageous to carry out the reaction at an elevated temperature, for example, at about 100-200°C. Ethylene, as is the hydrazine, is appropriately employed in excess to be certain that all of the water-insoluble palladium and gold compounds are converted into metallic palladium and gold. Palladium retention on the catalyst ranges from about 95% to about 100% by weight and gold retention ranges from about 85% to about 95% by weight of the original amount of palladium and gold impregnated on the carrier.

If the water-insoluble precious metal compounds are to be reduced to precious metals by gas phase reduction using ethylene, subsequent to the fixing step, the carrier is washed with distilled water to remove any anions, such as chloride ions, which are still contained on the carrier and freed from the impregnating solution. Washing is continued until all of the anions are removed from the carrier, approximately about 5 hours. To ensure substantially complete removal of the anions from the catalyst, the wash effluent is tested with silver nitrate after each washing until the silver nitrate test is negative, i.e., no conversion to silver chloride. The catalyst then is dried at temperatures not to exceed about 150°C under an inert atmosphere such as a continuous nitrogen flow.

If reduction of the water-insoluble precious metal compounds to free metal is achieved with hydrazine, washing to remove anionic species takes place subsequent to reduction. The resulting catalyst is washed with distilled water for about 5 hours or until the effluent from the wash tests negative with silver nitrate, i.e., no silver chloride formation. The carriers are dried at temperatures of from about 100°C to temperatures not to exceed about 150°C under an inert atmosphere of a continuous nitrogen flow.

Depending on the use for which the catalyst prepared according to the method of the present invention is intended, the catalyst also can be provided with customary additives. For example, additions of alkali metal acetates are advantageous when the catalyst is to be used for the preparation of unsaturated esters from olefins, oxygen and organic acids. In such a case, for example, the catalyst can be impregnated with an aqueous solution of potassium acetate, sodium acetate, lithium acetate, rubidium acetate or cesium acetate and then dried in an inert atmosphere of a continuous nitrogen flow.

The catalysts according to the present invention can be used with particular advantage in the preparation of vinyl acetate from ethylene, oxygen and acetic acid in the gas phase. Such catalysts according to the present invention which contain silica as the carrier material and additives of alkali metal acetates are particularly suitable. In the preparation of vinyl acetate, such catalysts are distinguished by high activity and selectivity. Moreover, catalysts of the present invention produce fewer heavy ends than catalysts prepared without an alkali metal borate fixing solution. Catalysts made in accordance with the method of the present invention have heavy end selectivity of below about 1.4%.

When vinyl acetate is prepared using the catalysts according to the present invention, a stream of gas, which contains ethylene, oxygen or air and acetic acid is passed over the catalyst. The composition of the gas stream can be varied within wide limits, taking into account explosive limits. For example, the molar ratio of ethylene to oxygen can be about 80:20 to about 98:2 and the molar ratio of acetic acid to ethylene can be about 100:1 to about 1:100 and the content of gaseous alkali metal acetate can be about 2-200 ppm, relative to the acetic acid employed. The gas stream also can contain other inert gases, such as nitrogen, carbon dioxide and/or saturated hydrocarbons. Reaction temperatures which can be used are elevated temperatures, preferably those in the range of about 150-220°C. The pressure employed can be a somewhat reduced pressure, normal pressure or elevated pressure, preferably a pressure of up to about 20 atmospheres gauge.

The following examples are intended to further illustrate the present invention but are not intended to limit the scope of the present invention.

### EXAMPLES I TO XII

Silica KA-160 catalyst carriers were provided by Sud Chemie. The carriers used in Examples I to VIII and Examples X to XII had a spherical shape and a diameter of about 7.0 mm. The carrier employed in Example IX also had a spherical shape but a diameter of about 5 mm. The carriers were divided into 12 (Examples I to XII) sets of 250 ml each.

Table 1 below sets forth the palladium and gold salts utilized to impregnate each catalyst, the fixing solution and the agent, either hydrazine or ethylene used to reduce the fixed palladium and gold compounds to free metal. All of the catalysts except for that of Example XI were impregnated with palladium and gold salt solutions having concentrations sufficient to provide about 6.6 gm/l of palladium and 3.0 gm/l gold on the carrier. In Example XI, separate applications of the palladium and gold salts were used at respective concentrations to provide 7.0 gm/l palladium and 4.0 gm/l gold on the catalyst.

The catalysts of Examples I-VII, IX, X and XII were prepared by utilizing a rotation immersion fixing step with either an alkali metal borate or alkali metal hydroxide fixing solution. The catalysts of Example VIII were prepared by the incipient wetness method both for impregnation of the gold and palladium salts as well as for the sodium hydroxide fixing solution. Example XI also utilized incipient wetness but separate palladium and gold impregnation steps and separate fixing steps were utilized as will be more fully explained below.

All of the catalysts which were prepared using a fixing step of roto immersion either in aqueous alkali metal borate or alkali metal hydroxide solution and were reduced with hydrazine, i.e., Examples I, III-V, VII, IX-X and XII were prepared as follows:

The silica carriers were impregnated with the specified palladium and gold salts as set forth in Table 1 by the incipient wetness method. Subsequent to impregnation with the specified palladium and gold salts, 250 ml of the impregnated supports were placed in a round bottom flask with about 300 ml of the fixing solution whether the alkali metal borate or alkali metal hydroxide as specified in Table 1. The respective volumes of the impregnated catalyst carriers and respective fixing solution equates to a molar ratio of borate to precious metals of about 2:1 and hydroxide to precious metals of about 1.2:1. The flasks were rotated for about 2 ½ hours at 5 rpm in a roto-evaporator (without vacuum). Subsequently, 4.4 gm of hydrazine monohydrate were added to the flasks and the flasks again rotated for 0.5 hours. After rotation, the carriers were allowed to stand for about 4 hours to complete the reduction process. The carriers were then washed with distilled water for about 5 hours until the wash effluent tested negative with silver nitrate. The water flow rate was about 200 cc/min. for each washing. After precious metal compounds were reduced to their metals and washed, the resulting catalysts were dried under nitrogen at a temperature of 100-150°C. The dried catalysts were then impregnated with an aqueous solution containing about 10 gms of potassium acetate at a solution volume equal to the carrier absorbtivity.

The catalyst preparations for Examples II and VI which both utilized a rotation immersion fixing step are the same as described above except that after the initial rotation immersion in the fixing solution, the catalysts were then washed with distilled water to remove the anionic species, air dried, and reduced with ethylene at a temperature of 150°C. The catalysts were then treated with potassium acetate as above described.

The catalysts of Example VIII were prepared according to the incipient wetness method wherein the impregnating solutions are poured over the catalysts and have a solution volume about equal to the dry absorbtivity of the carrier. The carriers were first impregnated with a sufficient amount of the specified palladium and gold salts as set forth in Table 1 to provide the desired Pd and Au loadings. The impregnated carriers were then air dried followed by fixing the water-soluble precious metal compounds as water-insoluble precious metal compounds by the incipient wetness method. Thus, an appropriate volume of an aqueous solution of sodium hydroxide was poured onto the impregnated carriers. The molar ratio of hydroxide to the precious metals was about 1.2:1.

The fixed water-insoluble precious metal compounds then were reduced to their metals with an excess of hydrazine monohydrate in aqueous solution again poured over the fixed catalyst. The molar ratio of hydrazine to precious metal was about 12:1. The carriers were allowed to stand for about 4 hours to insure that the reduction process was complete. Following reduction, the resulting catalysts were washed with distilled water for about 5 hours until the effluent tested negative with silver nitrate. The catalysts were dried at about 100-150°C under an inert atmosphere of nitrogen.

The catalysts then were impregnated with an aqueous solution containing about 10 gms of potassium acetate at a solution volume equal to the carrier absorbtivity.

The catalysts of Example XI were prepared as follows. The carriers were first impregnated by incipient wetness with a sufficient amount of the specified palladium salt as set forth in Table 1 to provide about 7.0 gm/l of palladium metal on the support. A water-insoluble palladium compound was precipitated on the carriers by adding 250 cc of the impregnated carriers to a round bottom flask containing about 283 ml of an aqueous solution of potassium borate such that the molar ratio of borate to palladium metal was about 2:1. The mixture then was rotated in a roto-evaporator (without vacuum) for about 2.5 hours at a rate of about 5 rpm.

The water-insoluble palladium compound fixed on the carriers was then reduced by addition of an aqueous solution containing about 4.94 gms of hydrazine monohydrate to the flask and rotating the mixture for about 0.5 hour and allowing the mixture to stand for about 4 hours to complete the reduction of the water-insoluble palladium compound to palladium metal on the carriers. The carriers were then dried in air at about 100°C.

The carriers containing the palladium metal were then impregnated by incipient wetness with an aqueous solution containing the specified gold salt (Table 1) in an amount sufficient to provide about 4.0 gm/l of gold metal on the catalyst and potassium borate. The molar ratio of borate to gold in solution was about 2:1. The mixture was allowed to stand over night, i.e., about 16 hours, to assure completion of the precipitation of the gold acid salt to the water-insoluble gold compound.

The fixed gold compound on the carriers was reduced with 283 ml of an aqueous solution containing 2.2 gms of hydrazine monohydrate to form gold metal on the carriers by roto immersion in which the catalysts were rotated for 0.5 hour in the aqueous hydrazine solution and then were allowed to stand in the solution for about 4 hours to assure complete reduction of the water-insoluble gold compound to gold metal. The resulting catalysts were washed with distilled water for about 5 hours or until the effluent from the wash tested negative with silver nitrate. The catalysts were then dried at a temperature not exceeding 150°C under an inert atmosphere of a continuous nitrogen flow.

The catalysts were then impregnated with a solution containing about 10 gms of potassium acetate at a solution volume equal to the carrier absorbtivity. The catalysts were again dried at a temperature not to exceed 100-150°C under an inert atmosphere.

### SYNTHESIS OF VINYL ACETATE WITH CATALYSTS FROM EXAMPLES I-XII

The catalysts prepared in the preceding examples were employed in the synthesis of vinyl acetate. About 60 ml of each type of catalyst prepared in the preceding examples were placed in separate chrome-nickel steel baskets. The temperature of each basket was measured by a thermocouple at both the top and bottom of each basket. Each reaction basket was placed in a Berty reactor and was maintained at a temperature which provided about 45% oxygen conversion with an electric heating mantle. A gas mixture of about 50 normal liters (measured at N.T.P.) of ethylene, about 10 normal liters of oxygen, about 49 normal liters of nitrogen and about 50 gm of acetic acid was caused to travel under pressure at about 12 atmospheres through each basket. Analysis of the products was accomplished by on-line gas chromatographic analysis combined with off-line liquid product analysis by condensing the product stream at about 10°C to obtain optimum analysis of the end products.

Table 2 below shows the results of vinyl acetate synthesis with catalysts prepared using an alkali metal borate to fix the palladium and gold water-soluble compounds on the catalyst carriers in contrast to catalysts prepared by fixing the palladium and gold water-soluble compounds with sodium hydroxide. Catalysts prepared with a fixing solution of alkali metal borate and, in particular, catalysts also reduced with hydrazine monohydrate in accordance with the method of the present invention (Examples I, V, VII, XI and XII) showed reduced heavy ends, 0.88, 0.77, 0.75, 0.844 and 0.788, and high activity, 2.22, 2.01, 1.99, 2.08 and 2.37, respectively, in contrast to catalysts prepared using sodium hydroxide as the fixing agent or reducing the precious metals with ethylene (see Table 1, Examples II to IV and Examples VI, VIII to X). While catalysts from Examples VIII and X prepared with a sodium hydroxide fix yielded reduced heavy ends, the activities of these catalysts were substantially below that of the catalyst formed with the borate fix.

**TABLE 1**

| | Catalyst Preparation Conditions | | |
|---|---|---|---|
| Catalyst¹ | Pd/Au salts³ | Fixing Agent | Reducing Agent |
| Example I | K₂PdCl₄/KAuCl₄ | Na Borate | Hydrazine |
| Example II | K₂PdCl₄/KAuCl₄ | Na Borate | Ethylene |
| Example III | Na₂PdCl₄/NaAuCl₄ | NaOH | Hydrazine |
| Example IV | Na₂PdCl₄/NaAuCl₄ | NaOH | Hydrazine |
| Example V | Na₂PdCl₄/NaAuCl₄ | Na Borate | Hydrazine |
| Example VI | Na₂PdCl₄/NaAuCl₄ | NaOH | Ethylene |
| Example VII | Na₂PdCl₄/NaAuCl₄ | Na Borate | Hydrazine |
| Example VIII | Na₂PdCl₄/NaAuCl₄ | NaOH | Hydrazine |
| Example IX² | Na₂PdCl₄/NaAuCl₄ | NaOH | Hydrazine |
| Example X | K₂PdCl₄/HAuCl₄ | KOH | Hydrazine |
| Example XI | K₂PdCl₄/⁴HAuCl₄ | K Borate | Hydrazine |
| Example XII | K₂PdCl₄/HAuCl₄ | K Borate | Hydrazine |

| | | | |
|---|---|---|---|
| 1. Silica carriers comprise 7mm spheres unless specified | | | |
| 2. Silica carrier comprise 5mm spheres | | | |
| 3. Salt solutions applied to provide 6.6 g/l Pd and 3.0 g/l Au on catalyst unless specified | | | |
| 4. Salt solutions applied to provide 7.0 g/l Pd and 4.0 g/l Au on catalyst | | | |

**TABLE 2**

| | Vinyl Acetate Production % Selectivity¹ | | | |
|---|---|---|---|---|
| Catalyst | Carbon Dioxide | Heavy Ends | Ethyl Acetate | Activity |
| Example I | 10.6 | 0.88 | 0.16 | 2.22 |
| Example II | 10.6 | 1.27 | 0.11 | 2.44 |
| Example III | 9.2 | 0.91 | 0.13 | 1.82 |
| Example IV | 9.1 | 0.95 | 0.12 | 1.79 |
| Example V | 10.8 | 0.77 | 0.18 | 2.01 |
| Example VI | 8.6 | 1.35 | 0.11 | 1.95 |
| Example VII | 10.8 | 0.75 | 0.17 | 1.99 |
| Example VIII | 13.9 | 0.51 | 0.09 | 1.04 |
| Example IX | 9.05 | 1.03 | 0.103 | 1.76 |
| Example X | 9.96 | 0.769 | 0.096 | 1.37 |
| Example XI | 11.6 | 0.844 | 0.096 | 2.08 |
| Example XII | 11.1 | 0.788 | 0.144 | 2.37 |

| | | | | |
|---|---|---|---|---|
| 1. Based on ethylene | | | | |

## Claims

1. A method of preparing an unsaturated ester catalyst comprising impregnating a catalyst carrier with a water-soluble palladium compound and a water-soluble gold compound; fixing the water-soluble palladium compound and the water-soluble gold compound to the carrier by forming a water-insoluble palladium compound and a water-insoluble gold compound with a fixing composition comprising an alkali metal borate solution; and reducing the water-insoluble palladium compound and the water-insoluble gold compound to palladium and gold metal to form a catalyst.

2. The method of claim 1, further comprising tumbling the impregnated carrier in the alkali metal borate solution by rotation to precipitate the water-soluble palladium compound to a water-insoluble palladium compound and to complete precipitation of the water-soluble gold compound to the water-insoluble gold compound.

3. The method of claim 2, wherein the impregnated carrier in the alkali metal borate solution is rotated from about 1 to about 10 rpm for about 0.5 hours to about 4 hours.

4. The method of claim 1, wherein the water-insoluble palladium compound and the water- insoluble gold compound are reduced to palladium metal and to gold metal with a reducing agent comprising ethylene or hydrazine.

5. The method of claim 4, wherein the reducing agent is hydrazine and a molar ratio of the hydrazine to the palladium and the gold metals is about 10:1 to about 15:1.

6. The method of claim 5, wherein the molar ratio of hydrazine to the palladium and the gold metals is about 12:1.

7. The method of claim 5, further comprising washing and drying the carrier after reducing the water-insoluble palladium and gold compound to palladium and gold metal.

8. The method of claim 7, wherein the drying is at a temperature of about 100°C in an inert atmosphere under a continuous nitrogen flow.

9. The method of claim 1, wherein the water-soluble palladium compound and the water-soluble gold compound impregnated on the carrier are fixed to the carrier as the water-insoluble palladium compound and the water-insoluble gold compound by pouring the alkali metal borate solution over the carrier at a volume equal to the dry absorbtivity of the carrier.

10. The method of claim 1, further comprising impregnating the carrier with an alkali metal acetate.

11. The method of claim 10, wherein the alkali metal acetate comprises potassium acetate, sodium acetate, lithium acetate, rubidium acetate or cesium acetate.

12. The method of claim 9, further comprising air drying the carrier after fixing the water-soluble palladium and gold compounds.

13. The method of claim 1, wherein the water-soluble palladium compounds comprise palladium chloride, sodium palladium chloride, potassium palladium chloride, palladium sulfate or palladium nitrate.

14. The method of claim 1, wherein the water-soluble gold compound comprises sodium or potassium salts of auric chloride or tetrachloroauric acid.

15. The method of claim 1, wherein the alkali metal borate comprises sodium tetraborate, sodium tetraborate pentahydrate, sodium tetraborate decahydrate, potassium tetraborate, potassium tetraborate pentahydrate or potassium tetraborate decahydrate.

16. The method of claim 1, wherein the amount of palladium metal added to the carrier comprises a concentration of about 1.0 gm/l to about 10.0 gm/l.

17. The method of claim 1, wherein the amount of gold metal added to the carrier comprises a concentration of from about 0.5 gm/l to about 10 gm/l.

18. The method of claim 1, wherein the amount of gold metal added to the carrier comprises about 3.0 gm/l and the amount of palladium metal comprises about 6.6 gm/l.

19. The method of claim 1, wherein the amount of gold metal added to the carrier comprises about 4.0 gm/l and the amount of palladium metal comprises about 7.0 gm/l.

20. The method of claim 1, wherein the carrier is silica.

21. The method of claim 1, wherein the water-soluble palladium compound is impregnated on the carrier and fixed on the carrier as the water-insoluble palladium compound then reduced to palladium metal prior to impregnating the carrier with the water-soluble gold compound.

22. The method of claim 21, further comprising the step of impregnating the carrier with a solution of the alkali metal borate and the water-soluble gold compound to fix the water-soluble gold compound on the carrier as the water-insoluble gold compound.

23. The method of claim 22, further comprising the step of reducing the water-insoluble gold compound to the gold metal.

24. The method of claim 1, wherein a molar ratio of the alkali metal borate to the palladium and gold metal in the water-soluble compounds is about 1.5:1 to about 2.5:1.

## Patentansprüche

1. Verfahren zur Herstellung eines Katalysators für ungesättigte Ester, umfassend das Imprägnieren eines Katalysatorträgers mit einer wasserlöslichen Palladiumverbindung und einer wasserlöslichen Goldverbindung; das Fixieren der wasserlöslichen Palladiumverbindung und der wasserlöslichen Goldverbindung auf dem Träger durch die Bildung einer wasserunlöslichen Palladiumverbindung und einer wasserunlöslichen Goldverbindung mit einer eine Alkalimetallborat-Lösung umfassenden Fixierungszusammensetzung und das Reduzieren der wasserunlöslichen Palladiumverbindung und der wasserunlöslichen Goldverbindung zu Palladium- und Goldmetall, wodurch ein Katalysator gebildet wird.

2. Verfahren nach Anspruch 1, weiterhin umfassend das Bearbeiten des imprägnierten Trägers in der Alkalimetallborat-Lösung in rotierenden Trommeln zum Ausfällen der wasserlöslichen Palladiumverbindung zu einer wasserunlöslichen Palladiumverbindung und zur Vervollständigung des Ausfällens der wasserlöslichen Goldverbindung zur wasserunlöslichen Goldverbindung.

3. Verfahren nach Anspruch 2, wobei der imprägnierte Träger in der Alkalimetallborat-Lösung mit etwa 1 bis etwa 10 U./min etwa 0,5 h bis etwa 4 h lang gedreht wird.

4. Verfahren nach Anspruch 1, wobei die wasserunlösliche Palladiumverbindung und die wasserunlösliche Goldverbindung mit einem Ethylen oder Hydrazin umfassenden Reduktionsmittel zu Palladiummetall und zu Goldmetall reduziert werden.

5. Verfahren nach Anspruch 4, wobei das Reduktionsmittel Hydrazin ist und das Stoffmengenverhältnis von Hydrazin zum Palladium- und Goldmetall etwa 10:1 bis etwa 15:1 beträgt.

6. Verfahren nach Anspruch 5, wobei das Stoffmengenverhältnis von Hydrazin zum Palladium- und Goldmetall etwa 12:1 beträgt.

7. Verfahren nach Anspruch 5, weiterhin umfassend das waschen und Trocknen des Trägers nach dem Reduzieren der wasserunlöslichen Palladium- und Goldverbindung zu Palladium- und Goldmetall.

8. Verfahren nach Anspruch 7, wobei das Trocknen bei einer Temperatur von etwa 100 °C in einer Inertatmosphäre unter einem kontinuierlichen Stickstoffstrom stattfindet.

9. Verfahren nach Anspruch 1, wobei die wasserlösliche Palladiumverbindung und die wasserlösliche Goldverbindung, die auf dem Träger imprägniert sind, als die wasserunlösliche Palladiumverbindung und die wasserunlösliche Goldverbindung am Träger fixiert werden, indem die Alkalimetallborat-Lösung mit einem Volumen, das gleich dem Trocken-Absorptionsvermögen des Trägers ist, über den Träger gegossen wird.

10. Verfahren nach Anspruch 1, weiterhin umfassend das Imprägnieren des Trägers mit einem Alkalimetallacetat.

11. Verfahren nach Anspruch 10, wobei das Alkalimetallacetat Kaliumacetat, Natriumacetat, Lithiumacetat, Rubidiumacetat oder Caesiumacetat umfaßt.

12. Verfahren nach Anspruch 9, weiterhin umfassend das Lufttrocknen des Trägers nach dem Fixieren der wasserlöslichen Palladium- und Goldverbindung.

13. Verfahren nach Anspruch 1, wobei die wasserlöslichen Palladiumverbindungen Palladiumchlorid, Natriumpalladiumchlorid, Kaliumpalladiumchlorid, Palladiumsulfat oder Palladiumnitrat umfassen.

14. Verfahren nach Anspruch 1, wobei die wasserlösliche Goldverbindung Natrium- oder Kaliumsalze von Gold(III)-chlorid oder Tetrachlorgoldsäure umfaßt.

15. Verfahren nach Anspruch 1, wobei das Alkalimetallborat Natriumtetraborat, Natriumtetraboratpentahydrat, Natriumtetraboratdecahydrat, Kaliumtetraborat, Kaliumtetraboratpentahydrat oder Kaliumtetraboratdecahydrat umfaßt.

16. Verfahren nach Anspruch 1, wobei die Menge des zum Träger zugegebenen Palladiummetalls eine Konzentration von etwa 1,0 g/l bis etwa 10,0 g/l umfaßt.

17. Verfahren nach Anspruch 1, wobei die Menge des zum Träger zugegebenen Goldmetalls eine Konzentration von etwa 0,5 g/l bis etwa 10 g/l umfaßt.

18. Verfahren nach Anspruch 1, wobei die Menge des zum Träger zugegebenen Goldmetalls etwa 3,0 g/l und die Menge des Palladiummetalls etwa 6,6 g/l umfaßt.

19. Verfahren nach Anspruch 1, wobei die Menge des zum Träger zugegebenen Goldmetalls etwa 4,0 g/l und die Menge des Palladiummetalls etwa 7,0 g/l umfaßt.

20. Verfahren nach Anspruch 1, wobei der Träger Siliciumdioxid ist.

21. Verfahren nach Anspruch 1, wobei die wasserlösliche Palladiumverbindung auf dem Träger imprägniert und als die wasserunlösliche Palladiumverbindung auf dem Träger fixiert wird, die dann zu Palladiummetall reduziert wird, bevor der Träger mit der wasserlöslichen Goldverbindung imprägniert wird.

22. Verfahren nach Anspruch 21, weiterhin umfassend die Stufe des Imprägnierens des Trägers mit einer Lösung des Alkalimetallborats und der wasserlöslichen Goldverbindung zum Fixieren der wasserlöslichen Goldverbindung als die wasserunlösliche Goldverbindung auf dem Träger.

23. Verfahren nach Anspruch 22, weiterhin umfassend die Stufe des Reduzierens der wasserunlöslichen Goldverbindung zum Goldmetall.

24. Verfahren nach Anspruch 1, wobei das Stoffmengenverhältnis von Alkalimetallborat zum Palladium- und Goldmetall in den wasserlöslichen Verbindungen etwa 1,5:1 bis etwa 2,5:1 beträgt.

## Revendications

1. Procédé de préparation d'un catalyseur pour la production d'esters insaturés, comprenant les étapes consistant à imprégner un support de catalyseur avec un composé de palladium hydrosoluble et un composé d'or hydrosoluble ; à fixer le composé de palladium hydrosoluble et le composé d'or hydrosoluble au support en formant un composé de palladium insoluble dans l'eau et un composé d'or insoluble dans l'eau avec une composition fixatrice comprenant une solution d'un borate de métal alcalin ; et à réduire le composé de palladium insoluble dans l'eau et le composé d'or insoluble dans l'eau en palladium métallique et en or métallique pour former un catalyseur.

2. Procédé suivant la revendication 1, comprenant en outre l'étape consistant à agiter au tambour le support imprégné dans la solution de borate de métal alcalin par rotation pour précipiter le composé de palladium hydrosoluble en un composé de palladium insoluble dans l'eau et pour précipiter totalement le composé d'or hydrosoluble en le composé d'or insoluble dans l'eau.

3. Procédé suivant la revendication 2, dans lequel le support imprégné dans la solution de borate de métal alcalin est agitée par rotation à une vitesse d'environ 1 à environ 10 tours/minute pendant un temps d'environ 0,5 heure à environ 4 heures.

4. Procédé suivant la revendication 1, dans lequel le composé de palladium insoluble dans l'eau et le composé d'or insoluble dans l'eau sont réduits en palladium métallique et en or métallique avec un agent réducteur comprenant de l'éthylène ou de l'hydrazine.

5. Procédé suivant la revendication 4, dans lequel l'agent réducteur consiste en hydrazine et le rapport molaire de l'hydrazine au palladium métallique et à l'or métallique est compris dans l'intervalle 10:1 à environ 15:1.

6. Procédé suivant la revendication 5, dans lequel le rapport molaire de l'hydrazine au palladium métallique et à l'or métallique est compris dans l'intervalle d'environ 12:1.

7. Procédé suivant la revendication 5, comprenant en outre l'étape consistant à laver et sécher le support après réduction du composé de palladium et du composé d'or insolubles dans l'eau en palladium métallique et en or métallique.

8. Procédé suivant la revendication 7, dans lequel le séchage est effectué à une température d'environ 100°C dans une atmosphère inerte sous un courant d'azote continu.

9. Procédé suivant la revendication 1, dans lequel le composé de palladium hydrosoluble et le composé d'or hydrosoluble imprégnant le support sont fixés au support sous forme de composé de palladium insoluble dans l'eau et de composé d'or insoluble dans l'eau en versant la solution de borate de métal alcalin sur le support en un volume égal à l'absorptivité à sec du support.

10. Procédé suivant la revendication 1, comprenant en outre l'imprégnation du support avec un acétate de métal alcalin.

11. Procédé suivant la revendication 10, dans lequel l'acétate de métal alcalin comprend l'acétate de potassium, l'acétate de sodium, l'acétate de lithium, l'acétate de rubidium ou l'acétate de césium.

12. Procédé suivant la revendication 9, comprenant en outre le séchage à l'air du support après avoir fixé le composé de palladium et le composé d'or hydrosolubles.

13. Procédé suivant la revendication 1, dans lequel les composés de palladium hydrosolubles comprennent le chlorure de palladium, le chlorure de sodium et de palladium, le chlorure de potassium et de palladium, le sulfate de palladium ou le nitrate de palladium.

14. Procédé suivant la revendication 1, dans lequel le composé d'or hydrosoluble comprend des sels de sodium ou de potassium de chlorure aurique ou d'acide tétrachloroaurique.

15. Procédé suivant la revendication 1, dans lequel le borate de métal alcalin comprend le tétraborate de sodium, le tétraborate de sodium pentahydraté le tétraborate de sodium décahydraté, le tétraborate de potassium, le tétraborate de potassium pentahydraté ou le tétraborate de potassium décahydraté.

16. Procédé suivant la revendication 1, dans lequel la quantité de palladium métallique ajoutée au support représente une concentration de 1,0 g/l à environ 10,0 g/l.

17. Procédé suivant la revendication 1, dans lequel la quantité d'or métallique ajoutée au support représente une concentration d'environ 0,5 g/l à environ 10 g/l.

18. Procédé suivant la revendication 1, dans lequel la quantité d'or métallique ajoutée au support représente une concentration d'environ 3,0 g/l et la quantité de palladium métallique représente une concentration d'environ 6,6 g/l.

19. Procédé suivant la revendication 1, dans lequel la quantité d'or métallique ajoutée au support représente une concentration d'environ 4,0 g/l et la quantité de palladium métallique représente une concentration d'environ 7,0 g/l.

20. Procédé suivant la revendication 1, dans lequel le support consiste en silice.

21. Procédé suivant la revendication 1, suivant lequel le composé de palladium hydrosoluble imprègne le support et est fixé sur le support sous forme de composé de palladium insoluble dans l'eau, puis est réduit en palladium métallique avant l'imprégnation du support avec le composé d'or hydrosoluble.

22. Procédé suivant la revendication 21, comprenant en outre l'étape consistant à imprégner le support avec une solution du borate de métal alcalin et du composé d'or hydrosoluble pour fixer le composé d'or hydrosoluble sur le support sous forme de composé d'or insoluble dans l'eau.

23. Procédé suivant la revendication 22, comprenant en outre l'étape consistant à réduire le composé d'or insoluble dans l'eau en or métallique.

24. Procédé suivant la revendication 1, dans lequel le rapport molaire du borate de métal alcalin au palladium métallique et à l'or métallique dans les composés hydrosolubles est compris dans l'intervalle d'environ 1,5:1 à environ 2,5:1.
